# EUROPEAN PATENT SPECIFICATION

(11) **EP 2 633 060 B1**
(45) Date of publication and mention of the grant of the patent: **03.10.2018**
(21) Application number: 11785617.9
(22) Date of filing: 25.10.2011
(51) Int. Cl.: C12P 7/10, C07K 14/33, C12N 9/42, C12N 15/56, C12N 15/62, C12N 9/24

(54) **COVALENT CELLULOSOMES AND USES THEREOF**
KOVALENTE ZELLULOSOMEN UND IHRE VERWENDUNG
CELLULOSOMES COVALENTS ET LEURS UTILISATIONS

(30) Priority: 25.10.2010 EP 10290573
(43) Date of publication of application: 04.09.2013
(73) Proprietor: TOTAL RAFFINAGE CHIMIE, 92400 Courbevoie (FR); LE CENTRE NATIONAL DE LA RECHERCHE SCIENTIFIQUE (CNRS), 75794 Paris, Cedex 16 (FR); Université d'Aix-Marseille, 13284 Marseille Cedex 07 (FR); L'Institut National des Sciences Appliquées (INSA), 31077 Toulouse Cedex 4 (FR)
(72) Inventor: FIEROBE, Henri-Pierre, 13009 Marseille (FR); CHANAL-VIAL, Angélique, Marseille 13012 (FR); RECEVEUR-BRÉCHOT, Véronique, 83270 Saint-Cyr-sur-Mer (FR); NOUAILLER, Matthieu, 13009 Marseille (FR)
(74) Representative: Paemen, Liesbet R.J.
(86) International application number: PCT/EP2011/068645
(87) International publication number: WO 2012/055863

(56) References cited:
- EP-A1- 2 192 177
- US-A1- 2009 035 811
- FIEROBE H.-P.: "Design and production of active cellulosome chimeras", THE JOURNAL OF BIOLOGICAL CHEMISTRY, vol. 276, no. 24, 15 June 2001 (2001-06-15), pages 21257-21261, XP002631541,
- DOI R.H. AND KOSUGI AKIHIKO: "cellulosomes:plant-cell-wall-degrading enzyme complexes", NATURE REVIEWS, MICCROBIOLOGY, vol. 2, 1 July 2004 (2004-07-01), pages 541-551, XP002631542,
- MINGARDON F ET AL: "Exploration of new geometries in cellulosome-like chimeras", APPLIED AND ENVIRONMENTAL MICROBIOLOGY, AMERICAN SOCIETY FOR MICROBIOLOGY, US, vol. 73, no. 22, 1 November 2007 (2007-11-01), pages 7138-7149, XP002518815, ISSN: 0099-2240, DOI: 10.1128/AEM.01306-07 [retrieved on 2007-09-28]

## Description

### FIELD OF THE INVENTION

The present invention relates to enzyme constructs with increased enzymatic activity. The present invention also relates to polynucleic acids encoding these constructs. The present invention further relates to cellulolytic (micro)organisms expressing and optionally secreting these constructs. Also, the present invention relates to methods for degrading lignocellulosic material which make use of the constructs according to the invention.

### BACKGROUND

Cellulosic and lignocellulosic materials are major constituents of plant biomass, and cellulose polymers found therein can provide a significant source of glucose or other fermentable mono- and oligosaccharides that can be used in the production of useful solvents, such as ethanol, acetone or butanol.

Cellulolytic micro-organisms have been an important source for the identification of enzymes and enzyme combinations capable of efficiently degrading cellulosic material. Most cellulolytic bacteria produce a large array of enzymes which interact in the degradation of cellulose. Cellulolytic anaerobic bacteria such as *Clostridium* or *Bacteroides* secrete cellulosomes, i.e. large multi-enzyme complexes to degrade the plant cell wall.

Their elevated activity, especially on the most recalcitrant and abundant plant polysaccharide, the crystalline cellulose, and their functioning has raised a fundamental interest for these sophisticated machineries. These cellulosomes also have an important potential as a tool for applied purposes, especially in the field of biofuels for the conversion of cellulosic material into fermentable sugars.

Natural bacterial cellulosomes are hybrid cellulosomes in which the assembly is based on the non-covalent interactions between cohesin modules, present on a backbone referred to as scaffoldin and a dockerin module borne by the enzymes. However, the recombinant expression of hybrid cellulosomes requires transformation of the host with multiple constructs which is less practical and affects efficiency.

Mingardon et al. (2007 Applied and Environmental Microbiology 73:7138-7149) discloses covalent cellulosomes wherein in a single polypeptide chain a CBM together with two or more catalytic modules are combined, however these catalytic modules are not interconnected by one or more spacers that have a length of more than 15 residues.

Accordingly, there is a need for methods and tools which allow a further improvement of the efficiency of degradation of cellulosic material either in vitro or by cellulolytic micro-organisms.

### SUMMARY OF THE INVENTION

The present invention is based on the observation that the cellulolytic activity of two or more naturally occurring cellulases (expressed by a host cell) can be significantly improved or increased when at least the catalytic modules of these cellulases are expressed in the form of a so-called "covalent cellulosome" (as further defined herein), wherein at least the catalytic modules of these cellulases are covalently linked or interconnected by one or more spacers which ensure a minimal distance between the two catalytic modules. More particularly, the spacer has a sequence of at least 40 amino acids. Accordingly, constructs are provided comprising at least two catalytic domains, which are connected by a covalent bond but which are separated by a spacer of at least 40 amino acids, and methods for use of such constructs.

In a first aspect, the present invention provides covalent cellulosomes comprising a binding domain comprising at least one carbohydrate binding module (CBM) of a cellulosomal scaffoldin protein fused to at least one hydrophilic X-module domain of a cellulosomal scaffolding protein, two or more polypeptides, each comprising at least a catalytic module of a cellulase enzyme of interest, and one or more spacers interconnecting the two or more polypeptides by a covalent bond, wherein each of the one or more spacers separating the polypeptides has a length of at least 40 residues. In further embodiments the spacers have a length of more than 120 residues.

In particular embodiments, the covalent cellulosomes according to the invention comprise at least one hydrophilic X-module domain of a cellulosomal scaffoldin protein, which may be but is not limited to at least one hydrophilic X-module domain selected from the group consisting of: the X1 module of CipA of *Clostridium thermocellum,* the X2 module of CipC of *Clostridium cellulolyticum,* the X2 module of CbpA *Clostridium cellulovorans,* the X2 module of CipA of *Clostridium josui,* and the X2 module of CipA of *Clostridium acetobutylicum.*

In particular embodiments, the cellulase enzyme is a bacterial enzyme. In further particular embodiments, the cellulase enzyme of interest is selected from the group consisting of the family of Cel 48, Cel 9, Cel 6 and/or Cel5 enzymes. More particularly the enzymes are selected from the group of:
a) a cellulase of *C. cellulolyticum* selected from the group comprising Cel48F, Cel9G, Cel9R, Cel9P, Cel9E, Cel9H, Cel9J, Cel9M, Cel8C, Cel5N, and Cel5A.
b) a cellulase of *S. degradans* strain 2-40 selected from the group comprising Cel9A, Cel9B, Cel5J, Cel5I, Cel5F, Cel5H, Cel5D, Cel5B, Cel9G, Cel5E, Cel5A, Cel5C and Cel6A. Most particularly, the catalytic modules are catalytic modules of Cel9G and Cel48F.

In certain particular embodiments, the covalent cellulosomes according to the invention comprise a carbohydrate binding type-3 module (CBM3), such as for instance a CBM3 module of type IIIa or the carbohydrate binding module of the CipC cellulosomal scaffolding protein of a *Clostridium cellulolyticum.*

In particular embodiments, the covalent cellulosomes according to the invention further comprise a signal peptide, such as for instance the signal peptide of the CipC scaffolding protein of *C. cellulolyticum,* or the signal peptide of the CipA scaffolding protein of *C. acetobutylicum.*

In particular embodiments the covalent cellulosomes of the present invention do not comprise a dockerin domain.

A further aspect of the invention provides polynucleic acids encoding the covalent cellulosomes according to the invention as well as vectors comprising such polynucleic acids and recombinant microorganisms expressing the covalent cellulosomes according to the invention.

In particular embodiments, the polynucleic acids encoding a covalent cellulosome as described herein further comprise a sequence ensuring secretion by a gram-negative bacterium.

In a further aspect, the present invention provides methods for producing a cellulolytic host cell, said method at least comprising the step of expressing the covalent cellulosome according to the invention in said host cell and ensuring secretion of said covalent cellulosome by said host cell.

In yet a further aspect, the present invention provides methods for degrading lignocellulosic material which involve expressing a covalent cellulosome according to the present invention in a host cell, separating the covalent cellulosome expressed by said host cell from said host cell, and contacting the lignocellulosic material with the covalent cellulosome expressed by said host cell, or which involve expressing the covalent cellulosome according to the present invention in a host cell and ensuring secretion of said covalent cellulosome by said host cell, and contacting said host cell with said lignocellulosic material.

In yet another aspect the invention provides methods for increasing the activity of two or more plant cell wall degrading enzymes expressed by a host cell, said methods comprising the step of expressing said plant cell wall degrading enzymes in the form of a covalent cellulosome according to the invention.

### Brief description of the drawings

The invention will now be described, inter alia with reference to the accompanying drawings, by way of example only, in which:
**Figure 1** provides the organization of different covalent cellulosomes as described herein.

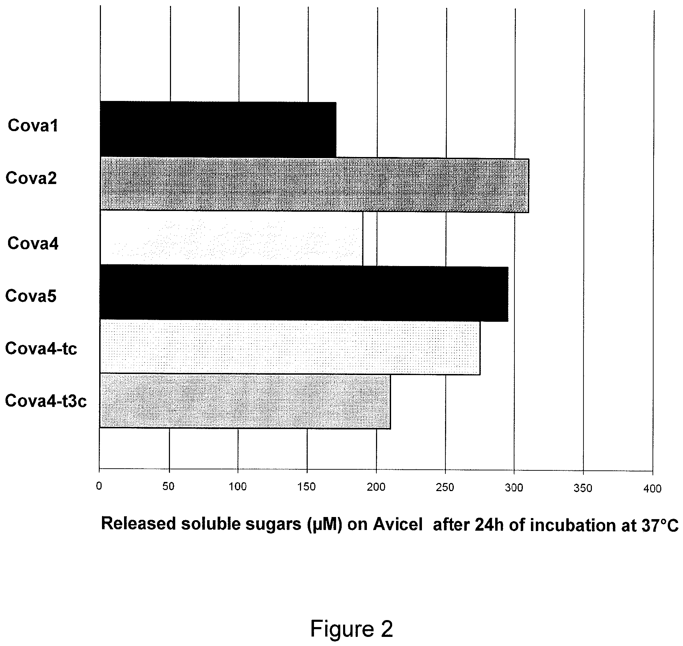

**Figure 2** demonstrates the total sugar production by of a number of covalent cellulosomes as defined in Figure 1 on Avicel 3,5 g/L over a period of 24 hours.

### DETAILED DESCRIPTION

The present invention will be described with respect to particular embodiments but the invention is not limited thereto but only by the claims. Any reference signs in the claims shall not be construed as limiting the scope.

Where an indefinite or definite article is used when referring to a singular noun e.g. "a" or "an", "the", this includes a plural of that noun unless something else is specifically stated.

The terms "comprising", "comprises" and "comprised of" as used herein are synonymous with "including", "includes" or "containing", "contains", and are inclusive or open-ended and do not exclude additional, non-recited members, elements or method steps. As used herein embodiments generally described as comprising particular features or steps include inter alia embodiments which essentially consist of or consist of the described features or steps.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "homologue" of a protein as used herein refers to a protein which has an amino acid sequence that has at least 30% identity, preferably at least 40%, 50%, 60%, 70%, 80% or 90% identity, most preferably at least 95% identity with a functional portion of the amino acid sequence of that protein. It should be understood that instead of % "identity", also the corresponding % "similarity" can be used to define homologues according to the invention.

The terms or definitions used herein are provided solely to aid in the understanding of the invention.

The present invention relates to constructs comprising two or more catalytic modules of cellulase enzymes for use in the degradation of (ligno)-cellulosic material. The constructs allow simultaneous production and use of different cellulase enzymes so as to improve efficiency of degradation. The constructs are referred to as covalent cellulosomes.

The term "covalent cellulosome" as used herein refers to a polypeptide chain comprising at least two catalytic modules of enzymes that are covalently bound or interconnected via one or more spacers. This is as opposed to the "naturally occurring cellulosomes", wherein catalytic modules of enzymes are anchored onto a polypeptide backbone based on a non-covalent interaction between cohesins present on the backbone and dockerin domains of the enzymes. More particularly, in the covalent cellulosomes of the present invention, the catalytic modules are linked by a covalent bond.

In a first aspect, covalent cellulosomes are provided comprising two or more polypeptides, each comprising at least a catalytic module of a cellulose degrading enzyme of interest, which are covalently linked or interconnected by one or more spacers. The spacers ensure a minimal distance between the catalytic modules which is beneficial to their combined activity. More particularly the spacers used herein have a length of at least 15 amino acids.

The covalent cellulosome described herein has particular improved properties compared to previously constructed hybrid cellulosomes, such as e.g. ease of production and secretion. The length of the spacer ensures that the activity of the catalytic modules is retained and even increased.

It has indeed been found by the present inventors that by separating the catalytic modules of the enzymes by a spacer having at least 15 amino acids, more particularly at least 20 amino acids, or at least 30, 40 or 50, in particular at least 100 or at least 105 amino acids, the activity of the catalytic modules can be increased, compared to when shorter spacers are used. More particularly it has been found that spacers of about 120 amino acids significantly increase the activity of the catalytic modules in the cellulosome described herein. Accordingly, the covalent cellulosomes described herein comprise at least two catalytic modules which are separated by a spacer of at least 15 amino acids.

The nature of the amino acids present in the spacer is not critical. However, in particular embodiments, the one or more spacer sequences comprise non-charged amino acids such as glycine, serine, cysteine, asparagine, tyrosine, glutamine, alanine, valine, proline, threonine, and more particularly glycine or serine. In further particular embodiments, the spacer is not glycosylated.

Examples of suitable spacer sequences can be found in bacterial cellulosomal scaffolding proteins such as but not limited to the peptide linkers of CipA of *C. acetobutylicum* (AE007606), CipC of *C. cellulolyticum* (U40345), CipA of *C. thermocellum* (X67406 or X67506), CbpA of *Clostridium cellulovorans* (M73817), CipA of *Clostridium josui* (AB004845). Where the sequences of these peptide linkers are less than 15 amino acids they can be used in the spacers described herein e.g. as combinations of two or more bacterial scaffoldin linkers, which can be the same or different. Accordingly it is envisaged that a spacer can comprise either one unique sequence or subsequent repetitive shorter sequences. In particular embodiments the covalent cellulosomes comprise a spacer comprising at least two linkers of bacterial scaffolding proteins.

The one or more spacer sequences for linking the at least two catalytic modules of plant cell wall degrading enzymes of interest comprised in a covalent cellulosome described herein may further comprise a sequence which is hydrophilic. More particularly the percentage of polar uncharged amino acids is relatively low, particularly below 60%, more particularly 50%, most particularly below 45%. Typically polar uncharged residues are S, T, N and Q.The linker may have less than 60% S and T residues. In particular embodiments the spacer sequence is a sequence encoding a hydrophylic X-module domain. In further particular embodiments the spacer comprises a hydrophilic X-module domain of the CipA scaffolding domain described in Sabathé et al. 2002, FEMS Microbiology Letters 217:15-22, Figure 4). In particular embodiments, the hydrophilic X-module domains are of bacterial origin.

It is envisaged that when more than two polypeptides are present in the covalent cellulosomes of the present invention these can be interconnected or separated by one or more spacers; accordingly, the spacers interconnecting the catalytic modules can be the same or different.

In particular embodiments the catalytic modules are directly linked by one ore more spacers as described herein, i.e. no other sequences are present between the catalytic modules.

The covalent cellulosomes described herein comprise two or more polypeptides each of which comprise a catalytic module of an enzyme, more particularly a plant cell wall degrading enzyme. The covalent cellulosomes described herein can comprise two polypeptides, each comprising a catalytic module of an enzyme, or the covalent cellulosomes can comprise more than two, particularly three, four, five or more polypeptides each comprising a catalytic module of an enzyme, more particularly a plant cell wall degrading enzyme.The polypeptides may essentially consist of a catalytic module of an enzyme. Alternatively, the polypeptides may contain a catalytic module of an enzyme and may further contain a "catalytic" or "helper" CBM such as CBM3c that targets carbohydrates other than crystalline cellulose like a CBM6.

As used herein, the term "plant cell wall degrading enzyme", refers to enzymes which catalyze the cleavage of cellulosic or lignocellulosic materials, and include but are not limited to cellulases, hemicellulases, cellobiohydrolases and other enzymes involved in breaking down plant cell wall polysaccharides into simple sugars such as glucose, xylose, arabinose, mannose and galactose.

In particular, the plant cell wall degrading enzymes are cellulases. The term "cellulase" or "cellulase enzyme" refers to a protein capable of degrading one or more cellulose substrates (*e.g.,* crystalline, semi-crystalline or amorphous cellulose) into simple sugars such as, but not limited to glucose, cellobiose, cellotriose, cellotetraose and/or cellopentaose. This term includes progressive and non-progressive cellulases. Progressive cellulase will continue to interact with a single polysaccharide strand, non-progressive cellulase will interact once then disengage and engage another polysaccharide strand. Explicitly, but not exclusively, included within the term "cellulase" or "cellulase enzyme" are enzymes categorized under the Enzyme Classification heading EC 3.2.1.4 enzymes, also called β-1,4-endoglucanases, cleave β-1,4-glycosidic linkages randomly along the cellulose chain, EC 3.2.1.91 enzymes also called cellobiohydrolases or exoglucanases which sequentially release cellobiose or glucose from one extremity of the cellulose chain, and EC 3.2.1.4/EC 3.2.1.91 enzymes also called endo-processive cellulases which display a mixed mode of action (both endo and exo glucanase). The term hemicellulase as used herein refers to a protein capable to degrade plant cell wall polysaccharides other than cellulose (e.g., xylan, mannan, xyloglucan, laminarin, β-1,3/β-1,4 glucans) into simple sugars such as, but not limited to xylose, mannose, arabinose and/or galactose. This term includes but is not restricted to xylanases (EC 3.2.1.8), mannanases (EC 3.2.1.78), xyloglucan hydrolases (EC 3.2.1.151), α-arabinofuranosidases (EC 3.2.1.55), feruloyl exterases (EC 3.1.1.73) acetyl xylan esterases (EC 3.1.1.72), β-xylosidases (EC 3.2.1.37), β-1,3/β-1,4 glucanases (EC 3.2.1.73.), β-1,3 glucanases (EC 3.2.1.39).

The origin of the plant cell wall degrading enzymes of interest or their catalytic modules used herein is not critical. The plant cell wall degrading enzymes of interest as referred herein may be of microbial origin, e.g. of fungal or bacterial origin. In further particular examples, the plant cell wall degrading enzymes of interest as referred to herein are of bacterial origin, for example from a bacterium of the genus *Alteromonadaceae,* e.g. *Saccharophagus degradans* strain 2-40, of the genus *Thermomonospora*, e.g., from *T. fusca,* of the genus *Cellulomonadaceae* e.g. *C. fimi,* of the genus *Clostridia,* e.g. from *Clostridium thermocellum, Clostridium cellulolyticum, Clostridium acetobutylicum, Clostridium cellulovorans, Clostridium josui.* In particular embodiments, the cellulase enzymes of interest include enzymes of fungal origin, for example from a fungus of the genus *Neocallimastigomycota,* e.g. from *N. patriciarum* or *Orpinomyces sp* strain PC-2.

Particular examples of plant cell wall degrading enzymes envisaged for use herein are members of the families of glycosyl hydrolases (GHF) capable of degrading cellulose, more particularly GHF5, 6, 7, 8, 9, 10, 12, 26, 44, 45, 48, 51, 61, and 74; The plant cell wall degrading enzymes may also be selected from the members of the GHF families 5, 6, 9 and 48. More particularly from the members of GHF families 9 and 48.

Particular examples of cellulases suitable for use in covalent cellulosomes according to the invention include but are not limited to:
- cellulases of *C. cellulolyticum* selected from the group comprising Cel48F, Cel9G, Cel9R, Cel9P, Cel9E, Cel9H, Cel9J, Cel9M, Cel8C, Cel5N, and Cel5A;
- cellulases of *C. thermocellum* selected from the group comprising Cel9D, Cel9J, CBH9A, Cel9H, Cel9K, Cel5E, Cel48S, Cel9F, Cel9N, Cel9Q, Cel50, Cel5B, Cel5G, Cel8A, Cel5C and Cel9I;
- cellulases of *S. degradans* strain 2-40 selected from the group comprising Cel9A, Cel9B, Cel5J, Cel5I, Cel5F, Cel5H, Cel5D, Cel5B, Cel9G, Cel5E, Cel5A, Cel5C and Cel6A.
- putative cellulases from *Pseudomonas* species ND 137 such as Acla.

In further particular examples, the plant cell wall degrading enzymes suitable for use in a covalent cellulosome according to the invention are the cellulases Cel48F and Cel9G of *C. cellulolyticum.*

In particular examples, at least one of the two or more catalytic modules of plant cell wall degrading enzymes of interest as referred to herein is the catalytic module of cellulase Cel5H of *S. degradans* strain 2-40.

A combination of two or more different enzymes can be envisaged for use herein. In particular, the different catalytic modules comprised in the covalent cellulosomes described herein have a complementary activity in the degradation of ligno-cellulosic material.

More particularly it is envisaged that the cellulosomes described herein may be used to complement commercial enzyme cocktails, by providing for the missing activities therein. For instance, it has been found that many commercial cocktails based of fungal enzymes do not contain activity corresponding to the activity of the enzymes of families GHF9 or GHF48.

The covalent cellulosomes described herein are further typically characterized by the presence of a binding domain comprising a carbohydrate binding module (CBM) and a hydrophilic module.The carbohydrate binding module and hydrophilic module may be present at the N-terminus of the covalent cellulosome. Accordingly, the covalent cellulosomes described herein may comprise a sequence corresponding to the formula CBM-X-CAT1-CAT2, wherein CBM corresponds to a carbohydrate binding module, X stands for hydrophilic domain, CAT1 and CAT2 stand respectively for a first and a second catalytic module (whereby CAT1 and CAT2 may be the same or different), "-" corresponds to a connecting sequence and "-" represents a spacer. These additional elements are described more in detail hereafter.

In the context of the present invention, the terms "carbohydrate binding module", "carbohydrate binding molecule", "carbohydrate binding protein" and "carbohydrate binding domain" are used interchangeably herein and refer to a protein or functional part (i.e. capable of binding carbohydrates), or a homologue thereof, which is capable of binding a polysaccharide substrate, such as e.g. cellulose. The carbohydrate binding modules (CBMs) are functionally independent and frequently occur in nature in association with proteins and complexes involved in biomass breakdown, such as cellulases and cellulosomes.

The carbohydrate binding modules are carbohydrate binding modules of cellulosomal scaffolding proteins. The term "cellulosomal scaffolding protein" or "scaffoldin" as used herein refers to a scaffolding protein comprised in a (naturally occurring, i.e. hybrid) cellulosome.

The covalent cellulosomes described herein may also comprise a carbohydrate binding module derived from an enzyme (such as a cellulase enzyme), wherein the CBM module is similar to a carbohydrate binding module from a cellulosomal scaffolding protein. Carbohydrate-binding modules have been classified into more than 40 families according to sequence homology. Several cellulolytic enzymes share a conserved region of about 150 amino acid residues, named the CBM3 domain. The CBM3 domain has been classified in three different subtypes, termed family IIIa, IIIb and IIIc.

In particular examples, the covalent cellulosomes described herein comprise a carbohydrate binding module which is a CBM3 module, i.e. a carbohydrate binding type-3 module. In a further particular example, the covalent cellulosome described herein comprises a carbohydrate binding module which is a CBM3 module of type IIIa or a CBM3 module of type IIIb. The carbohydrate binding modules of family IIIa bind to crystalline cellulose.

Particular examples of carbohydrate binding modules that can be comprised in the covalent cellulosomes according to the invention include but are not limited to carbohydrate binding modules of cellulosomal scaffolding proteins selected from the group consisting of cellulosome integrating protein A (CipA) of *Clostridium thermocellum,* cellulosome integrating protein C (CipC) of *Clostridium cellulolyticum,* cellulose binding protein A (CbpA) of *Clostridium cellulovorans,* and cellulosome integrating protein A (CipA) of *Clostridium acetobutylicum.*

A particular example of a carbohydrate binding module comprised in binding domain of a covalent cellulosome according to the invention is the carbohydrate binding module of the scaffolding protein CipC of *Clostridium cellulolyticum* or a homologue thereof. More particularly, also envisaged herein are covalent cellulosomes as described above, wherein said at least one carbohydrate binding module is a homologue of the scaffolding protein CipC of *Clostridium cellulolyticum* and comprises a polypeptide having at least 30% identity, preferably at least 40%, 50%, 60%, 70%, 80% or 90% identity, most preferably at least 95% identity with the carbohydrate binding module of the scaffolding protein CipC of *Clostridium cellulolyticum.*

The structure of the covalent cellulosome described herein may comprises the sequence represented by CBM-CM1-L(n)-CM2, wherein CBM is a carbohydrate binding module, CM1 and CM2 represent a first and a second catalytic module (which can be the same or different) and L(n) represents one or more linkers, more particularly one or more linkers comprising a hydrophilic module.

Irrespective of the nature of the spacer separating the different catalytic modules, the binding domains present in the covalent cellulosomes described herein may be further characterized by the presence of at least one X module, more particularly a hydrophilic module, and preferably a hydrophilic module of a cellulosomal scaffolding protein. More particularly the covalent cellulosomes described herein are characterized by the presence of an X module present between the CBM and the first catalytic module.

The terms "hydrophilic domain", "hydrophilic module" and "X module" are used herein as synonyms and refer to a hydrophilic domain of a cellulosomal scaffolding protein. In particular embodiments, the X-module is an X-module of a mesophilic *Clostridium* cellulosomal scaffolding protein.
Thus, the X module comprised in a covalent cellulosome described herein may be of bacterial origin, preferably from a bacterium of the genus *Clostridia,* more particularly a mesophilic *Clostridium,* e.g. from *Clostridium cellulolyticum, Clostridium acetobutylicum, Clostridium josui* or *Clostridium cellulovorans.* It shall be noted that X modules found in *C. acetobutylicum, C. cellulolyticum, C. cellulovorans, C. josui* may be referred to as "X2" modules, while X modules found in *C. thermocellum* may be called "X1" modules. Particular examples of X modules comprised in a covalent cellulosome according to the invention comprise but are not limited to hydrophilic domains of cellulosomal scaffolding proteins selected from the group comprising cellulosome integrating protein A (CipA) of *Clostridium thermocellum,* cellulosome integrating protein C (CipC) of *Clostridium cellulolyticum,* cellulose binding protein A (CbpA) of *Clostridium cellulovorans,* cellulosome integrating protein A (CipA) of *Clostridium josui* and cellulosome integrating protein A (CipA) of *Clostridium acetobutylicum.* In particular embodiments of the invention, the at least one X module comprised in the covalent cellulosome according to the invention is the X2 module of the scaffolding protein CipC of *Clostridium cellulolyticum.*

In particular embodiments of the invention, the at least one X module comprised in the covalent cellulosome according to the invention is an X2 module of the scaffolding protein CipA of *Clostridium acetobutylicum.*

In further examples, the at least one X-module comprised in the covalent cellulosome described herein is a module homologous to the X modules described herein. More particularly, the X-module can be homologous to an X module described herein and a polypeptide having at least 30% identity, preferably at least 40%, 50%, 60%, 70%, 80% or 90% identity, most preferably at least 95% identity with the hydrophilic module of the scaffolding protein CipC of *Clostridium cellulolyticum* or of the scaffolding protein CipA of *Clostridium acetobutylicum.*

The CBM module and the at least one X module of the covalent cellulosomes may originate from the same cellulosomal scaffolding protein, or they may originate from different cellulosomal scaffolding proteins.

The covalent cellulosome described herein may comprise the structure X-CBM-CM1-L(n)-CM2, wherein X is a hydrophilic module, CBM is a carbohydrate binding module, CM1 and CM2 represent a first and a second catalytic module (which can be the same or different) and L(n) represents one or more linkers.

It will be understood by the skilled person that the CBM module and/or hydrophilic module present as a binding domain in the covalent cellulosome described herein are typically present 5' of the set of polypeptides comprising the catalytic modules. However, the CBM module and/or hydrophilic module may also be placed 3' of the set of polypeptides in the constructs described herein. The covalent cellulosomes according to the invention typically also comprise further peptide connecting sequences for connecting the carbohydrate binding domain and the at least one X-modules to the at least two catalytic modules of cellulase enzymes of interest. The nature and/or origin of these peptide connecting sequences is not the subject of the present invention. They may or may not correspond to the spacer sequences described herein. These connecting sequences may for example be typical linker sequences as known by the person skilled in the art. These connecting sequences present in a cellulosome according to the present invention are shorter than the spacer sequence separating the two catalytic modules. In further particular embodiments, the one or more connecting sequences have a length of less than 100 amino acids, more particularly, less than 45 amino acids. In further particular embodiments these connecting sequences have a length of less than 15 amino acid sequences.

According to particular embodiments of the invention, the covalent cellulosome does not comprise a dockerin domain and/or a cohesion domain. Accordingly, this, in addition to the length of the spacer used between two catalytic domains, further differentiates the covalent cellulosomes of the present invention from the constructs described in the prior art comprising one or more dockerin domains for use in combination with scaffoldins comprising one or more cohesion domains, such as those described by Mingardon et al. (2007, Appl. Environ. Microbiol. 73(22):7138-7149).

In particular embodiments of the invention, as is described more in detail below, the covalent cellulosomes are envisaged for production and optionally secretion by a microbial host. Where secretion by the microbial host is envisaged, the covalent cellulosomes according to the present invention may further comprise a sequence corresponding to a secretion signal sequence. This secretion signal sequence can be linked to the CBM domain such that the signal sequence is covalently linked thereto. In particular embodiments, the DNA coding for the signal sequence is at the 5' end of the construct, more particularly 5' end of the CBM module encoding DNA.

It shall be appreciated that suitable signal sequences may depend on the type of micro-organism in which secretion is desired.

Depending on the nature of the host cell, distinct signal sequences may be required.

More particularly, secretion is envisaged by a microbial host such as *Bacillus subtilis.* This organism is widely used in industrial processes. By means of example and not limitation, secretion in *Bacillus subtilis,* may be achieved using one of the signal peptides (twin-arginine (RR/KR-type) signal peptides, secretory (Sec-type) signal peptides, lipoprotein signal peptides, pseudopilin-like signal peptides, a signal peptide of a pheromone or bacteriocin, identified in *Bacillus subtilis* (Tjalsma H et al. Microbiology and Molecular Biology Reviews, June 2004, p. 207-233, Vol. 68, No. 2), or more particularly the signal sequence of *B. subtilis* alkaline protease (AprE exemplary sequence: Genbank acc. no. CAA74536, seq. version 1 revised on June 26^{th} 1997). In further particular embodiments secretion in *Bacillus subtilis* is envisaged by using the signal sequence of *Bacillus licheniformis* serine alkaline protease (*subC*) signal sequence (exemplary sequence: Genbank acc. no. AAU23708, seq. version 4 revised on April 26^{th} 2007), or the signal peptide from the *B. amyloliquefaciens* α-amylase (AmyQ exemplary sequence: Genbank acc. no. AAA22191, seq. version 1 revised on April 26^{th} 1993).

By further means of example and not limitation, secretion in Gram-positive bacteria, and in particular in *Clostridium* such as *C. acetobutylicum,* may be achieved using the signal sequence of the Cel5A precursor polypeptide of *C. cellulolyticum* (exemplary sequence: Genbank acc. no. AAA51444, seq version 1 revised on October 31, 1994), or of the CipC precursor scaffolding protein of *C. cellulolyticum* (exemplary sequence: Genbank acc. no. AAC28899, seq. 15 version 2 revised on December 5, 2005), or of the CipA precursor scaffolding protein of *C. acetobutylicum* (exemplary sequence: Genbank acc. no. AAK78886, seq. version 1 revised on January 19, 2006).

It shall also be appreciated that native (or homologous, endogenous) signal peptides of polypeptides to be expressed by the micro-organisms as taught herein may be employed, insofar as they are functional in said micro-organisms. Hence, by means of example, secretion of Cel48F or Cel9G of *Clostridium cellulolyticum* may be achieved using the CipC scaffolding protein of *C. cellulolyticum.* Similarly, secretion of Cel5H or related polypeptides in a heterologous organism may be achieved using the endogenous or homologous secretion signal sequence of the Cel5H precursor polypeptide.

In further embodiments, secretion by gram negative bacteria may be envisaged. Different secretion mechanisms have been described which ensure secretion of proteins in gram-negative bacteria. These include, but are not limited to the *Escherichia coli* alkaline phosphatase (phoA) signal peptide, fusion with the bacterial OsmY gene, and others.

It will be understood to the skilled person that the sequences ensuring secretion of the cellulosomes according to the present invention, do not need to be comprised in the constructs of the cellulosome or may be designed to be removed from the cellulosome after secretions.

In further embodiments secretion by yeast may be envisaged. For secretion by yeast, the use of yeast secretion signals may be envisaged. Different yeast secretion signals have been described including aF (Brake, 1990, Methods Enzymol., 185, 408-421; Hitzeman et al., 1990, Methods Enzymol., 185, 421-440.), KILM1 (Skipper et al., 1985, Science, 230, 958-960), PHO1 (Laroche et al., 1994, Bio/Technology, 12, 1119-1124) and SUC2 (Hitzeman et al., 1990, above). Alternatively, native signal peptides of the enzymes to be expressed can be used. By means of example and not limitation, secretion by *Saccharomyces cerevisiae* may be envisaged by using the mating factor alpha (MFA exemplary sequence: Genbank acc. no. AAA34778) prepro leader sequence.

Further provided herein are polynucleic acids encoding a covalent cellulosome as described herein. The synthesis of different enzymes as one covalent construct allows for a reduction in the number of transformations and thus facilitates production.

In particular a polynucleic acid is provided encoding a covalent cellulosome as described herein, i.e. comprising
- a binding domain comprising at least one carbohydrate binding module (CBM) of a cellulosomal scaffoldin protein (as defined herein) fused to at least one hydrophilic (X-module) domain of a cellulosomal scaffolding protein
- two or more polypeptides, each comprising at least a catalytic module of a plant cell wall degrading enzyme of interest (as defined herein), and
- one or more spacers (as defined herein) interconnecting the two or more polypeptides; The sequence encoding the spacer described herein encodes a protein which separates the two catalytic modules by a distance comparable to the distance ensured by a peptide of at least 15 residues. In particular the sequence encoding the spacer may encode a sequence of a length of more than 15 residues.

The polynucleic acids described herein thus comprise sequences encoding the different subunits as described herein.

In particular a polynucleic acid is provided encoding a covalent cellulosome as described herein, wherein the two or more catalytic modules are separated by spacers having a length of at least 15, more particularly at least 40 residues.

The nucleic acid sequences encoding the covalent cellulosomes according to the present invention typically contain suitable regulatory or control sequences for expression in a host cell. The terms "regulatory sequences" and "control sequence" used herein are to be taken in broad context and refer to regulatory nucleic acid sequences capable of driving and/or regulating expression of the sequences to which they are ligated (covalently linked) and/or operably, linked. The control sequences may differ depending upon the intended host organism and upon the nature of the sequence to be expressed. For expression of a protein in prokaryotes, the control sequences generally include a promoter, a ribosomal binding site, and a terminator. In eukaryotes, control sequences generally include promoters, terminators and, in some instances, enhancers, and/or 5' and 3' untranslated sequences. The term 'control sequence' is intended to include, at a minimum, all components necessary for expression, and may also include additional advantageous components. According to particular embodiments of the present invention, the control sequence is operable in a bacterium, which can be a gram-positive or a gram-negative bacterium. The term "control sequence" encompasses a promoter or a sequence capable of activating or enhancing expression of a nucleic acid molecule in a host cell.

Further provided herein are vectors comprising a polynucleic acid as described above and expression constructs to facilitate the introduction of the covalent cellulosomes described herein into a host cell and/or to facilitate expression and/or facilitate maintenance of the polynucleotide sequence encoding a covalent cellulosome as described herein. The expression constructs may be inserted into a plasmid or a vector, which may be commercially available.

By the term "vector" as used herein is meant a polynucleotide molecule, preferably a DNA molecule derived, for example, from a plasmid, bacteriophage, or plant virus, into which a polynucleotide can be inserted or cloned. A vector preferably contains one or more unique restriction sites and can be capable of autonomous replication in a defined host cell, or be integrable with the genome of the defined host such that the cloned sequence is reproducible. The choice of the vector will typically depend on the compatibility of the vector with the host cell into which the vector is to be introduced.

According to particular embodiments of the present invention, the expression construct is a bacterial expression vector, suitable for transformation into bacteria and suitable for maintenance and expression of a covalent cellulosome according to the present invention in a transformed bacterial cell. According to a further embodiment, the vector is a yeast transformation vector. The application thus also relates to vectors comprising any of the nucleic acids described above.

The vector ordinarily carries one or more replication sites as well as marker sequences, which are capable of providing phenotypic selection in transformed cells.

According to particular embodiments of the invention, the sequences encoding the covalent cellulosomes of the present invention are linked to a constitutive promoter. Examples of constitutive promoters suitable for the constructs and methods according to the present invention include but are not limited to the CaMV35S promoter, GOS2, actin promoter, ubiquitin promoter, thiolase promoter. According to another embodiment of the invention, the vectors comprise an inducible promoter. Examples of inducible promoters suitable for the constructs and methods according to the present invention include but are not limited to the lac promoter or xylose inducible promoter. Optionally, the present expression vectors will also contain sequences necessary for the termination of transcription and for stabilizing the mRNA, and may thus contain one or more transcription termination sequences. The term "transcription termination sequence" encompasses a control sequence at the end of a transcriptional unit, which signals 3' processing and termination of transcription. Additional regulatory elements, such as transcriptional or translational enhancers, may be incorporated in the expression construct. The expression constructs of the invention may further include an origin of replication that is required for maintenance and/or replication in a specific cell type. One example is when an expression construct is required to be maintained in a bacterial cell as an episomal genetic element (e.g. plasmid or cosmid molecule). Preferred origins of replication include, but are not limited to the f1-ori, colE1 ori, and Gram+ bacteria origins of replication.

The expression construct may optionally comprise a selectable marker gene. As used herein, the term "selectable marker gene" includes any gene, which confers a phenotype on a cell in which it is expressed to facilitate the identification and/or selection of cells which are transfected or transformed with an expression construct of the invention. Suitable markers may be selected from markers that confer antibiotic or herbicide resistance or visual markers. Examples of selectable marker genes include genes encoding neomycin phosphotransferase (nptll), hygromycin phosphotransferase (hpt) or Basta. Further examples of suitable selectable marker genes include resistance genes against ampicillin (AmpR), tetraclyclin (TcR), kanamycin (KanR), phosphinothricin, and chloramphenicol or thiamphenicol (CAT). Other suitable marker genes provide a metabolic trait, for example manA. Visual marker genes may also be used and include for example beta-glucuronidase (GUS), luciferase and Green Fluorescent Protein (GFP).

Construction of suitable vectors containing one or more of the above listed components and including the desired coding and control sequences employs standard ligation techniques. Isolated plasmids or nucleic acid fragments are cleaved, tailored, and religated in the form desired to generate the plasmids required.

The invention further provides recombinant microorganisms expressing and optionally secreting the covalent cellulosome according to the invention. Accordingly, the present invention provides host cells comprising a polynucleic acid (or a vector) encoding a covalent cellulosome according to the present invention.

The term "host cell" refers to those cells capable of growth in culture and capable of expressing a polynucleic acid as defined herein and thus capable of producing and optionally secreting a covalent cellulosome as defined herein. The host cells described herein encompass in vitro cell cultures and include prokaryotic cells. Particular examples relate to micro-organisms. Particular examples of host cells which may be used in accordance with the present invention include bacterial cells and yeast cells.

It shall be noted that the term "host cell" is intended to include all forms of the life cycle of the host cell such as spores.

In particular embodiments, the host cells envisaged in the context of the invention are bacterial cells, yeast or fungal cells. The selection of the host cell may be determined by the envisaged application. In particular embodiments, the host cell is a cell which allows high level recombinant expression of the covalent cellulosome, such as, but not limited to a *Saccharomyces cerevisiae, Candida lipolytica, E. coli, Arthrobacter, Rhodotorula glutinins, Acinetobacter, Pichia pastoris, Botryococcus braunii, Vibrio furnissii, Micrococcus leuteus, Stenotrophomonas maltophilia, Lactococcus lactis* or *Bacillus subtilis* cell, e.g., an *Arthrobacter AK 19, Acinetobacter* sp. strain M-I, *E. coli B, E. coli C, E. coli K* or *E. coli W* cell. In other embodiments, the recombinant cell is a cyanobacteria cell, e.g., a *Synechocystis sp.* or *Synechococcus elongatus* cell. In still other embodiments, the recombinant cell is a plant, animal or human cell. Alternatively, the recombinant cell is a microorganism cell from a bacterium, yeast, or filamentous fungus.

Methods for ensuring high level recombinant expression of proteins such as the covalent cellulosomes of the present invention in host cells are known in the art.

In particular embodiments, the host cells are capable (either naturally or as a result of genetic modification) of producing other products starting from the reaction product of the covalent cellulosome activity. In particular embodiments, the host cells are engineered to convert simple sugars into products such as acids (e.g. citric, lactic, proprionic or acetic acid (or ethyl acetate)), alkanes, biopolymers, polymer precursors, vitamins and electricity.

In further particular embodiments, the covalent cellulosome may be expressed in a solventogenic host. A solventogenic host refers to a host cell that is, either naturally or as a result of genetic modification, capable of converting sugars or acids into solvent, such as, but not limited to ethanol or butanol.

In particular embodiments, the host cell is capable of expressing the covalent cellulosomes according to the present invention. This can be ensured by the use of signal peptides or other secretion mechanisms or can be ensured by the natural mechanisms of the host cells. Typically, the covalent cellulosomes secreted by a host cell according to the present invention can ensure degradation of a substrate present in the medium of the host cell, such that the host cell is referred to as cellulolytic. Accordingly, a further aspect relates to methods for producing a cellulolytic organism, which comprise the step of expressing the covalent cellulosomes described herein in a host cell and ensuring secretion thereof by said host cell.

A further aspect relates to methods for degrading lignocellulosic or cellulosic material, which methods comprise contacting the lignocellulosic or cellulosic material with a covalent cellulosome as described herein.

Degradation can be envisaged by contacting the cellulosic material with recombinant covalent cellulosomes. More particularly it is envisaged that further to recombinant production of the covalent cellulosomes of the present invention, the cellulosomes are separated from the host cell (whereby the cellulosomes are recovered either as a secretion product or by chemical or mechanical disruption of the host cell). The covalent cellulosomes may thus either be present in medium or in isolated form. This type of degradation is referred to as *"in vitro",* as it is not performed in the presence of the host cell.

In particular embodiments, the plant cell wall degrading covalent cellulosome is recovered from the host organism by chemical or mechanical disruption of the cell, optionally followed by separation techniques which remove the protein from cellular debris. Such methods are known in the art.

In particular, a method for degrading (ligno-)cellulosic material is provided, comprising contacting said ligno-cellulosic material with a covalent cellulosome as described herein, wherein the degradation of (ligno-)cellulosic material is increased relative to a degradation in the absence of the covalent cellulosome. In such a method, the ligno-cellulosic material is contacted with the cellulosome described herein under conditions which ensure degradation of said lignocellulosic material. Such conditions are known in the art.

The methods of the present invention may be used in the context of the microbial production of products such as, but not limited to acids (e.g. citric, lactic, proprionic or acetic acid (or ethyl acetate)), alkanes, biopolymers, polymer precursors, vitamins and electricity. Accordingly, methods are provided for the microbial production of a product based on degradation of ligno-cellulosic material, which method comprises comprising contacting said ligno-cellulosic material with a covalent cellulosome according to the present invention, wherein the degradation products of said (ligno-)cellulosic material is further converted into the product of interest either *in vitro* or *in vivo* (e.g. by contacting with a micro-organism capable of converting the degradation products into the product of interest).Degradation of (ligno-)cellulosic material may be envisaged by contacting the host cell with the cellulosic material and ensuring secretion of the covalent cellulosome into the medium. This may require the use of specific secretion signals or secretion mechanisms, such as those described herein above. Such degradation methods are referred to herein as *"ex vivo"* as they are performed outside the cell but in the presence of the host cell. Accordingly, methods are provided for degrading (ligno-)cellulosic material which comprise contacting said material with one or more covalent cellulosomes according to the present invention, or a composition comprising one or more covalent cellulosomes according to the invention.

In particular, a method is provided for increasing degradation of (ligno-)cellulosic material in a host cell, comprising: transforming the host cell with a nucleotide sequence so that the host cell expresses or over-expresses a covalent cellulosome according to the present invention, wherein the degradation of (ligno-)cellulosic material in the host cell has been increased relative to a cell that has not been transformed. In such a method, the host cells may be harvested and lysed to obtain the covalent cellulosomes that have been produced. Alternatively, the host cell is transformed with a nucleotide sequence encoding a transport protein or the covalent cellulosome is provided with a signal peptide and the host cell releases the covalent cellulosomes extracellularly.

The methods described herein may be used in the context of the microbial production of products such as, but not limited to acids (e.g. citric, proprionic or acetic acid (or ethyl acetate)), alkanes, biopolymers, polymer precursors, vitamins and electricity. Accordingly, methods are provided for the microbial production of a product based on degradation of (ligno-)cellulosic material, which method comprises transforming the host cell with a nucleotide sequence so that the host cell expresses or over-expresses a covalent cellulosome according to the present invention, wherein the degradation of (ligno-)cellulosic material in the host cell has been increased relative to a cell that has not been transformed and said product is produced by said microbial host cell in amounts relative to the amount of material degraded by said host cell.

The present invention envisages the advantageous use of cellulosomes rather than isolated enzymes as it allows for bringing different enzymes into close contact with the plant cell wall polysaccharide material simultaneously. This allows e.g. for complementary enzymes to work efficiently in the degradation of the substrate. The selection of combinations of enzymes may be inspired on the naturally occurring enzyme combinations in cellulosomes or in hyperthermophilic anaerobic bacteria.

For instance, the combination of glycoside hydrolase 48 (GH48) and GH9 catalytic modules generate a very active complex on crystalline cellulose.

In the in vitro examples, the use of the covalent cellulosomes is envisaged both as such, as in combination with individual enzymes or other enzyme preparations also referred to as "enzyme cocktails", such as, but not limited to Cellic™ enzyme products from Novozymes®, such as CTec™, designed to breakdown pretreated biomass into fermentable sugars. Accordingly, enzyme cocktails are provided comprising one or more covalent cellulosomes as described herein. The enzyme cocktail may comprise a covalent cellulosome comprising the catalytic module of a GH48 and a GH9 enzyme.

The methods described herein typically involve contacting the covalent cellulosomes described herein with the lignocellulosic material under conditions which allow cellulose degradation. Conditions for cellulose degradation are known to the skilled person and may vary depending on the nature of the cellulose material (*e.g.,* crystalline, semi-crystalline or amorphous cellulose) or hemicellulose.

The invention will now be further illustrated by the following particular non-limiting examples.

### EXAMPLES

### 1. Synthesis of covalent cellulosomes

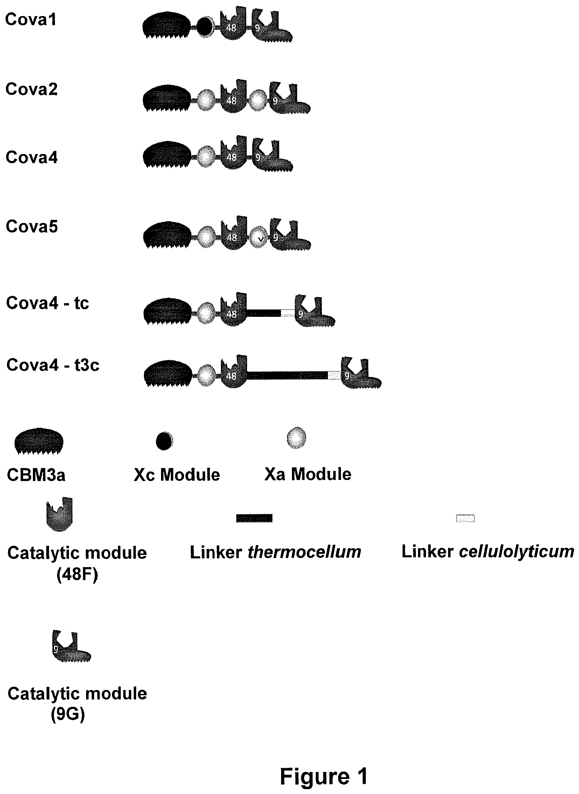

The covalent cellulosomes illustrated in Figure 1 were synthesized by overlap extension PCR using suitable primers and genomic DNA from *C. cellulolyticum, C. acetobutylicum* and *C. thermocellum.* The resulting genes were cloned in pET22b(+) vector at Ndel and Xhol sites and the resulting vector was checked by sequencing. *E. coli* BL21(DE3) strain was used as the production strain. After transformation, the cells were grown at 37°C in flasks containing Luria Bertani medium supplemented with ampicillin flasks until OD600 reaches 1.5. Induction with IPTG at 100 µM was performed overnight at 18 °C. Cells were subsequently harvested by centrifugation and disrupted using a French press. The crude extract was loaded on a Ni-NTA resin, and the protein of interest was subsequently eluted using 100 mM Imidazolium. The protein sample was concentrated in Vivaspin centricons, and loaded on Resource Q anion exchanger (GE healthcare), to achieve purification. The protein of interest was eluted using a linear gradient of 0-1M NaCl.

Cova1 is a covalent cellulosome comprising a CMB3 module, one X module (Xc) of CipC of *C. cellulolyticum,* and the catalytic modules 48F and 9G separated by a spacer of 11 amino acids (corresponding to the linker between the catalytic module of Cel48F and its native dockerin module).

Cova2 is a covalent cellulosome comprising a CMB3 module separated from 48F by the first X module (Xa) obtained from the CipA protein of *C. acetobutylicum* and wherein the catalytic domains 48F and 9G are separated by a spacer comprising another Xa module. Cova4 is a covalent cellulosome comprising a CMB3 module, the first X-module (Xa) of CipA of *C. acetobutylicum* and wherein the catalytic domains 48F and 9G are separated by a spacer comprising 11 amino acids.

Cova5 is a covalent cellulosome comparable to Cova 4 except that the spacer separating Cel48F and Cel9G comprises an Xa' module.

Cova4tc is a covalent cellulosome comparable to Cova 4 except that the spacer separating Cel48F and Cel9G is replaced by the inter-cohesins linker of the scaffoldin of *C. thermocellum* (37 residues) followed by the inter-cohesins linker of the scaffoldin of *C. cellulolyticum* (11 residues), resulting in a new spacer of 48 amino acids.

In Cova4t3c, the original spacer in Cova4 separating the two catalytic modules is replaced by three « *thermocellum* » linkers and one « *cellulolyticum* » linker, such that the spacer separating the catalytic modules is 122 amino acids long.

### 2. Activity of covalent cellulosomes on Avicel

Purified covalent cellulosomes were mixed at a final concentration of 0.1 µM with 3.5 g/L Avicel (Ph 101, Fluka) in 20 mM Tris-Maleate pH 6.0, 1 mM CaCl₂, 0.01% NaN3 and incubated at 37°C under mild shaking (60 rpm). At specific intervals, aliquots were pipetted, centrifuged and the soluble sugars in the supernatants were analyzed using the Park and Johnson method, or in a Dionex ICS3000 apparatus equipped with pulsed amperometric detection.

| Name | Length of spacer or module between the catalytic modules | Soluble sugars released on Avicel (3,5 g/L) in 24 hrs |
|---|---|---|
| Cova1 | 11 aa | 170 µM |
| Cova2 | 119 aa | 310 µM |
| Cova4 | 11 aa | 195 µM |
| Cova5 | 84 aa | 295 µM |
| Cova4tc | 48 aa | 275 µM |
| Cova4t3c | 122 aa | 210 µM |

The results allow a classification of the different covalent cellulosomes in a hierarchy based on their specific activity on this crystalline substrate: Cova2 ≥ Cova5 > Cova4 ≥ Cova1.

The cellulolytic activity of the different covalent cellulosomes was further compared over a time period of 24 hrs. As can be deduced from this figure, the introduction of a spacer of 48 aa in Cova4 (Cova4-tc) results in an increase of activity which is almost comparable to that of Cova2.

### 3. Secretion of covalent cellulosomes by C. acetobutylicum

The engineered gene encoding Cova4 was fused by overlap extension PCR with the DNA encoding the signal sequence of CipC from *C. cellulolyticum,* and the resulting amplicon was cloned in pSOS952 shuttle vector at BamHI and Narl sites. After methylation of the vector, the plasmid was used to electrotransform *C. acetobutylicum* according to standard procedure. Positive clones were selected on RCA-erythromycin containing plates incubated at 37°C anaerobically in a jar. The cells were subsequently grown anaerobically in 10 mL 2YT-cellobiose (5 g/L) rich medium until OD₆₂₀ reaches 3.0. The cells were harvested by centrifugation and the supernatant was concentrated 100 times in a Vivaspin cell. Cell sample and concentrated supernatant were subjected to polyacrylamide electrophoresis gel in denaturing conditions. A western blot analysis was performed using antisera raised against Cel48F and Cel9G. The covalent cellulosome was detected in both the cellular fraction and the concentrated supernatant.

## Claims

1. A covalent cellulosome comprising
- a binding domain comprising at least one carbohydrate binding module (CBM) of a cellulosomal scaffolding protein fused to at least one hydrophilic X-module domain of a cellulosomal scaffolding protein ;
- two or more polypeptides, each comprising at least a catalytic module of a cellulase enzyme of interest, and
- one or more spacers interconnecting the two or more polypeptides by a covalent bond;
wherein each of said one or more spacers has a length of at least 40 residues.

2. The covalent cellulosome according to claim 1, wherein said one or more spacers are **characterized by** one or more of the following features:
- they have less than 60% polar uncharged residues; and
- they comprise a hydrophylic X-module domain, preferably a hydrophilic X-module domain of the CipA scaffolding domain of *C. acetobutylicum.*

3. The covalent cellulosome according to any one of claims 1 or 2, wherein said cellulase enzyme is a bacterial enzyme.

4. The covalent cellulosome according to any one of claims 1 to 3, wherein said cellulase enzyme of interest is selected from the group consisting of
a) a cellulase of C. cellulolyticum selected from the group comprising Cel48F, Cel9G, Cel9R, Cel9P, Cel9E, Cel9H, Cel9J, Cel9M, Cel8C, Cel5N, and Cel5A.
b) a cellulase of S. degradans strain 2-40 selected from the group comprising Cel9A, Cel9B, Cel5J, Cel5I, Cel5F, Cel5H, Cel5D, Cel5B, Cel9G, Cel5E, Cel5A, Cel5C and Cel6A.

5. The covalent cellulosome according to any one of claims 1 to 4, wherein said at least one carbohydrate binding module is a carbohydrate binding type-3 module (CBM3), preferably a CBM3 module of type IIIa or the carbohydrate binding module of the CipC cellulosomal scaffolding protein of a Clostridium cellulolyticum.

6. The covalent cellulosome according to any one of claims 1 to 5, wherein said at least one hydrophilic X-module domain is selected from the group consisting of:
- the X1 module of CipA of Clostridium thermocellum
- the X2 module of CipC of Clostridium cellulolyticum
- the X2 module of CbpA Clostridium cellulovorans
- the X2 module of CipA of Clostridium josui
- The X2 module of CipA of Clostridium acetobutylicum.

7. The covalent cellulosome according to any one of claims 1 to 6, which further comprises a signal peptide.

8. A polynucleic acid encoding a covalent cellulosome according to any one of claims 1 to 7.

9. The polynucleic acid according to claim 8, which further comprises a sequence ensuring secretion by a gram-negative bacterium.

10. A vector comprising a polynucleic acid according to claim 8 or 9.

11. A recombinant microorganism expressing the covalent cellulosome according to any one of claims 1 to 7.

12. A method for producing a cellulolytic host cell, said method comprising the step of expressing the covalent cellulosome according to any one of claims 1 to 7 in said host cell and ensuring secretion of said covalent cellulosome by said host cell.

13. A method for degrading lignocellulosic material, which method comprises
- expressing the covalent cellulosome according to any one of claims 1 to 7 in a host organism,
- separating said covalent cellulosome expressed by said host cell from said host cell, and
- contacting the lignocellulosic material with said covalent cellulosome expressed by said host cell.

14. A method for degrading lignocellulosic material, which method comprises
- expressing the covalent cellulosome according to any one of claims 1 to 7 in a host organism and ensuring secretion of said covalent cellulosome by said host cell, and
- contacting said host cell with said lignocellulosic material.

15. A method for increasing the cellulolytic activity of two or more cellulases expressed by a host cell, said method comprising expressing said cellulases in the form of a covalent cellulosome according to any one of claims 1 to 7.

## Patentansprüche

1. Kovalentes Cellulosom, umfassend
- eine Bindungsdomäne, die mindestens ein Kohlenhydratbindungsmodul (CBM) eines cellulosomalen Gerüstproteins, fusioniert an mindestens eine hydrophile X-Modul-Domäne eines cellulosomalen Gerüstproteins, umfasst,
- zwei oder mehr Polypeptide, die jeweils mindestens ein katalytisches Modul eines Cellulaseenzyms von Interesse umfassen, und
- einen oder mehrere Spacer, die die zwei oder mehr Polypeptide durch eine kovalente Bindung miteinander verbinden,
wobei jeder des einen oder der mehreren Spacer eine Länge von mindestens 40 Resten besitzt.

2. Kovalentes Cellulosom nach Anspruch 1, wobei der eine oder die mehreren Spacer durch eines oder mehrere der folgenden Merkmale gekennzeichnet ist/sind:
- sie weisen weniger als 60% polare ungeladene Reste auf und
- sie umfassen eine hydrophile X-Modul-Domäne, vorzugsweise eine hydrophile X-Modul-Domäne der CipA-Gerüstdomäne von *C. acetobutylicum.*

3. Kovalentes Cellulosom nach einem der Ansprüche 1 oder 2, wobei das Cellulaseenzym ein bakterielles Enzym ist.

4. Kovalentes Cellulosom nach einem der Ansprüche 1 bis 3, wobei das Cellulaseenzym von Interesse aus der Gruppe ausgewählt ist, bestehend aus
a) einer Cellulase von *C. cellulolyticum,* ausgewählt aus der Gruppe, die Cel48F, Cel9G, Cel9R, Cel9P, Cel9E, Cel9H, Cel9J, Cel9M, Cel8C, Cel5N und Cel5A umfasst,
b) einer Cellulase des *S. degradans*-Stamms 2-40, ausgewählt aus der Gruppe, die Cel9A, Cel9B, Cel5J, Cel5I, Cel5F, Cel5H, Cel5D, Cel5B, Cel9G, Cel5E, Cel5A, Cel5C und Cel6A umfasst.

5. Kovalentes Cellulosom nach einem der Ansprüche 1 bis 4, wobei das mindestens eine Kohlenhydratbindungsmodul ein Kohlenhydratbindungs-Typ-3-Modul (CBM3), vorzugsweise ein CBM3-Modul des Typs IIIa oder das Kohlenhydratbindungsmodul des cellulosomalen Gerüstproteins CipC eines *Clostridium cellulolyticum,* ist.

6. Kovalentes Cellulosom nach einem der Ansprüche 1 bis 5, wobei die mindestens eine hydrophile X-Modul-Domäne aus der Gruppe ausgewählt ist, bestehend aus:
- dem X1-Modul von CipA von *Clostridium thermocellum*
- dem X2-Modul von CipC von *Clostridium cellulolyticum*
- dem X2-Modul von CbpA von *Clostridium cellulovorans*
- dem X2-Modul von CipA von *Clostridium josui*
- dem X2-Modul von CipA von *Clostridium acetobutylicum.*

7. Kovalentes Cellulosom nach einem der Ansprüche 1 bis 6, das weiterhin ein Signalpeptid umfasst.

8. Polynukleinsäure, die ein kovalentes Cellulosom nach einem der Ansprüche 1 bis 7 codiert.

9. Polynukleinsäure nach Anspruch 8, die weiterhin eine Sequenz umfasst, die die Sekretion durch ein Gramnegatives Bakterium sicherstellt.

10. Vektor, der eine Polynukleinsäure nach Anspruch 8 oder 9 umfasst.

11. Rekombinanter Mikroorganismus, der das kovalente Cellulosom nach einem der Ansprüche 1 bis 7 exprimiert.

12. Verfahren zur Herstellung einer cellulolytischen Wirtszelle, wobei das Verfahren den Schritt des Exprimierens des kovalenten Cellulosoms nach einem der Ansprüche 1 bis 7 in der Wirtszelle und das Sicherstellen der Sekretion des kovalenten Cellulosoms durch die Wirtszelle umfasst.

13. Verfahren zum Abbau von Lignocellulose-Material, wobei das Verfahren Folgendes umfasst
- Exprimieren des kovalenten Cellulosoms nach einem der Ansprüche 1 bis 7 in einem Wirtsorganismus,
- Abtrennen des von der Wirtszelle exprimierten kovalenten Cellulosoms von der Wirtszelle und
- Inkontaktbringen des Lignocellulose-Materials mit dem von der Wirtszelle exprimierten kovalenten Cellulosom.

14. Verfahren zum Abbau von Lignocellulose-Material, wobei das Verfahren Folgendes umfasst
- Exprimieren des kovalenten Cellulosoms nach einem der Ansprüche 1 bis 7 in einem Wirtsorganismus und Sicherstellen der Sekretion des kovalenten Cellulosoms durch die Wirtszelle und
- Inkontaktbringen der Wirtszelle mit dem Lignocellulose-Material.

15. Verfahren zum Erhöhen der cellulolytischen Aktivität von zwei oder mehr Cellulasen, die von einer Wirtszelle exprimiert werden, wobei das Verfahren das Exprimieren der Cellulasen in Form eines kovalenten Cellulosoms nach einem der Ansprüche 1 bis 7 umfasst.

## Revendications

1. Cellulosome covalent comprenant
- un domaine de liaison comprenant au moins un module de liaison aux glucides (CBM) d'une protéine d'échafaudage cellulosomique fusionnée à au moins un domaine de module X hydrophile d'une protéine d'échafaudage cellulosomique ;
- deux polypeptides ou plus, chacun comprenant au moins un module catalytique d'une enzyme cellulase d'intérêt, et
- un ou plusieurs espaceurs interconnectant les deux polypeptides ou plus par une liaison covalente ;
dans lequel chacun desdits un ou plusieurs espaceurs a une longueur d'au moins 40 résidus.

2. Cellulosome covalent selon la revendication 1, dans lequel lesdits un ou plusieurs espaceurs sont **caractérisés par** une ou plusieurs des caractéristiques suivantes :
- ils ont moins de 60 % de résidus non chargés polaires ; et
- ils comprennent un domaine de module X hydrophile, de préférence un domaine de module X hydrophile du domaine d'échafaudage CipA de *C. acetobutylicum.*

3. Cellulosome covalent selon l'une quelconque des revendications 1 ou 2, dans lequel ladite enzyme cellulase est une enzyme bactérienne.

4. Cellulosome covalent selon l'une quelconque des revendications 1 à 3, dans lequel ladite enzyme cellulase d'intérêt est choisie dans le groupe constitué de
a) une cellulase de C. cellulolyticum choisie dans le groupe comprenant Cel48F, Cel9G, Cel9R, Cel9P, Cel9E, Cel9H, Cel9J, Cel9M, Cel8C, Cel5N et Cel5A,
b) une cellulase de S. degradans souche 2-40 choisie dans le groupe comprenant Cel9A, Cel9B, Cel5J, Cel5I, Cel5F, Cel5H, Cel5D, Cel5B, Cel9G, Cel5E, Cel5A, Cel5C et Cel6A.

5. Cellulosome covalent selon l'une quelconque des revendications 1 à 4, dans lequel ledit au moins un module de liaison aux glucides est un module de liaison aux glucides de type 3 (CBM3), de préférence un module CBM3 de type IIIa ou le module de liaison aux glucides de la CipC protéine d'échafaudage cellulosomique de Clostridium cellulolyticum.

6. Cellulosome covalent selon l'une quelconque des revendications 1 à 5, dans lequel ledit au moins un domaine de module X hydrophile est choisi dans le groupe constitué des :
- module X1 de CipA de Clostridium thermocellum
- module X2 de CipC de Clostridium cellulolyticum
- module X2 de CbpA de Clostridium cellulovorans
- module X2 de CipA de Clostridium josui
- Module X2 de CipA de Clostridium acétobutylicum.

7. Cellulosome covalent selon l'une quelconque des revendications 1 à 6, qui comprend en outre un peptide signal.

8. Acide polynucléique codant pour un cellulosome covalent selon l'une quelconque des revendications 1 à 7.

9. Acide polynucléique selon la revendication 8, qui comprend en outre une séquence assurant la sécrétion par une bactérie Gram négatif.

10. Vecteur comprenant un acide polynucléique selon la revendication 8 ou 9.

11. Micro-organisme recombinant exprimant le cellulosome covalent selon l'une quelconque des revendications 1 à 7.

12. Procédé de production d'une cellule hôte cellulolytique, ledit procédé comprenant l'étape d'expression du cellulosome covalent selon l'une quelconque des revendications 1 à 7 dans ladite cellule hôte et assurant la sécrétion dudit cellulosome covalent par ladite cellule hôte.

13. Procédé de dégradation de matériau lignocellulosique, ledit procédé comprenant
- l'expression du cellulosome covalent selon l'une quelconque des revendications 1 à 7 dans un organisme hôte,
- la séparation dudit cellulosome covalent exprimé par ladite cellule hôte à partir de ladite cellule hôte, et
- la mise en contact du matériau lignocellulosique avec ledit cellulosome covalent exprimé par ladite cellule hôte.

14. Procédé de dégradation de matériau lignocellulosique, ledit procédé comprenant
- l'expression du cellulosome covalent selon l'une quelconque des revendications 1 à 7 dans un organisme hôte et l'assurance de la sécrétion dudit cellulosome covalent par ladite cellule hôte, et
- la mise en contact de ladite cellule hôte avec ledit matériau lignocellulosique.

15. Procédé pour augmenter l'activité cellulolytique de deux cellulases ou plus exprimées par une cellule hôte, ledit procédé comprenant l'expression desdites cellulases sous la forme d'un cellulosome covalent selon l'une quelconque des revendications 1 à 7.
